# EUROPEAN PATENT APPLICATION

(11) **EP 2 047 822 A2**
(43) Date of publication of application: **15.04.2009**
(21) Application number: 08166000.3
(22) Date of filing: 07.10.2008
(51) Int. Cl.: A61F 2/01

(54) **Embolic protection device having a filter frame with integral distal strain relief**

(30) Priority: 11.10.2007 US 870725
(71) Applicant: Cordis Corporation, Miami Lakes, FL 33014 (US)
(72) Inventor: Wijeratne, Lalith Hiran, Cooper City, FL FL33328-5168 (US); Mosel, Brian James, Dublin, CA CA 94568 (US); Rincon, Cesar Alberto, Sunrise, FL FL33322 (US)
(74) Representative: Belcher, Simon James

(57) **Abstract**

An embolic protection device provides a filter membrane for removing emboli from a fluid flowing in a vessel of a patient. The filter membrane is mounted on a filter frame having an integrated strain relief mechanism that provides strain relief and kink resistance in the transition between the relatively stiff filter and the flexible distal tip of the guidewire that is used to guide the filter into the treatment area. The strain relief mechanism is one or more cuts through the distal end of the filter frame. The cut pattern, pitch and variation of the pitch from one end to the other provides a way of fine-tuning the strain relief to a wide variety of filter frames, guidewires and flexible distal guidewire tips.

## Description

The present invention relates to an embolic protection device for use in percutaneous procedures and, more particularly, to an improved embolic protection device for filtering bodily fluids flowing through a vessel of a patient by providing a strain relief between the filter frame and the distal end of the device to provide easier access of the device distal to the treatment site.

Various surgical and intravenous treatments have been developed for treating and/or removing obstructions such as plaque in stenosed regions of vessels of a patient. In balloon angioplasty, a balloon placed on the distal end of a catheter is inflated, usually with a fluid, in order to dilate the stenosed region of the vessel thereby improving fluid flow. In addition a stent may be placed in the stenosed region of the vessel in order to reduce the occurrence and severity of restenosis of the vessel. In an atherectomy or thrombectomy, a rotating blade or other similar cutting device can be used to remove plaque in a stenosed region. Surgery may also be used to remove plaque and improve fluid flow in the vessel. All of these treatments result in the production of small particles of plaque or other materials resulting in emboli that can travel in the direction of fluid flow in the vessel and block smaller vessels downstream of the stenosis resulting in stroke, tissue damage or other problems.

In order to prevent such an occurrence various embolic protection devices have been suggested that place a filter distal to the stenosed region before and/or during the angioplasty, stenting, atherectomy or other emboli producing procedure. One type of filter is a woven wire mesh filter. Another type of filter is a polymeric material with filter pores placed on or around a filter frame often made of a self-expanding shape-memory metal such as a nickel-titanium alloy known as nitinol. These filter elements are mounted on the distal end of a guidewire or other elongated member which is used to place the filter into the vessel of the body through an external opening in the body containing the vessel. In some systems a guidewire is first inserted into the lumen of the vessel and is directed past a lesion or other area of interest. After insertion of the guidewire a filter element is pushed along the wire past the lesion or other area of interest where it is deployed. For example, United States Patent No. 6,179,859 issued to Bates et al. and entitled "Emboli Filtration System and Method of Use" describes a filter that is restrained in a delivery sheath until it is placed in the vessel.

In another type of embolic protection system the guidewire and filter element are simultaneously inserted into the lumen of the vessel and the filter element is directed past the lesion or other area of interest where it is deployed. For example, in United States Patent No, 6,468,291 issued to Bates et al. and entitled "Emboli Filtration System Having Integral Strut Arrangement and Methods of Use" such an embolic protection device is disclosed.

In an embolic protection device filter elements are usually mounted on one or more collars either at the distal end, the proximal end or both. These collars are then either fixed to the guidewire or are allowed to travel between one or more stops placed on the guidewire to allow longitudinal movement of the guidewire in relation to the filter. The proximal and distal collars of the filter basket may be allowed to rotate on the guidewire to provide rotational movement of the guidewire relative to the filter.

In an embolic protection device, the guidewire and/or the delivery sheath restraining the embolic protection device is pushed through the vessel. Ideally, the performance of the embolic protection device and guidewire system should be the same as a guidewire alone. In many embolic protection devices when the filter is captured within its delivery sheath the device is relatively stiff at the distal end of the filter. The guidewire, however, usually has a very flexible coil tip so as to be atraumatic and maneuverable. The transition between the stiff distal end of the filter and the flexible coil tip of the guidewire is prone to kinking. It would, therefore, be desirable to provide an embolic protection device that could reduce kinking at this transition in order to provide a more maneuverable and useful device.

It would also be desirable to have an embolic protection device wherein the strain relief could be easily changed or "tuned" in order to accommodate differences between various filters, guidewires and atraumatic guidewire tips.

The filter frame and filter have a larger diameter than the central guidewire core thereby causing a step transition between the guidewire core and the filter frame at the distal end. This step transition can cause problems within the vessel during implementation of the device. Furthermore, it would be desirable to have an embolic protection device in which there is a smooth transition between the atraumatic distal tip and the filter frame.

The present invention generally relates to an embolic protection device for capture and removal of emboli from a fluid flowing in a vessel. More particularly, the invention relates to the filter frame, particularly the transition of the distal end of the filter frame to the guidewire or core wire that is used to guide the embolic protection device into place. The filter frame has a means for providing strain relief near the transition from the filter and the guidewire core. A portion of the distal section or distal collar of the filter frame may be cut in various configurations in order to provide an integrated strain relief element in this transition area.

The strain relief means or strain relief element may also be tapered so as to provide a smooth transition between the guidewire or core wire and the filter frame so as to avoid a problematic step transition. The strain relief means or strain relief element may be covered with a sleeve, such as a polymeric sleeve, to create an atraumatic transition.

The strain relief means or strain relief element of the present invention can be "tuned" by changing the cut pattern, the pitch of the cut and how the pitch varies to the distal end of the filter frame. Additionally, because the strain relief element is integral to the filter frame there is less chance of the strain relief element breaking away from the embolic protection device and thereby causing a hazard in the patient.

The strain relief means or strain relief element may be a single spiral cut or double spiral cut pattern or may have an interlocking castellated pattern.

Embodiments of the invention will now be described by way of example with reference to the accompanying drawings, in which:
FIG. 1 is an elevational view of a first embodiment of an embolic protection device in accordance with the present invention.
FIG. 2 is a perspective view of a flexed filter frame and guidewire core of the embolic protection device of FIG. 1 in accordance with the present invention.
FIG. 3 is an elevational view of a portion of the filter frame of an embolic protection device having a spiraling integrated strain relief in accordance with the present invention.
FIG. 4 is an elevational view a portion of an embolic protection device having integrated strain relief with a polymeric sleeve thereon.
FIG. 5 is an elevational view of the filter frame of an embolic protection device in accordance with the present invention.
FIG. 6 is an elevational view of the distal end of the filter frame of an embolic protection device in accordance with the present invention.
FIG. 7 is a cut pattern for a 4-8-4 filter frame having spiraling integrated strain relief in accordance with the present invention.
FIG. 8 is a cut pattern for a 6-12-6 filter frame having a spiraling slotted integrated strain relief in accordance with the present invention.
FIG. 9 is a cut pattern for a 4-8-4 filter frame having a spiraling slotted integrated strain relief in accordance with the present invention.
FIG. 10 a cut pattern for the distal end of a 4-8-4 filter frame having spiraling integrated strain relief in accordance with the present invention.
FIG. 11 a cut pattern for the distal end of a 4-8-4 filter frame having spiraling integrated strain relief in accordance with the present invention.
FIG. 12 is a cut pattern for the distal end of a 4-8-4 filter frame having spiraling castellated integrated strain relief in accordance with the present invention.
FIG. 13 is a partial cross-sectional and elevational view of an embodiment of a filter system with deployment sleeve in accordance with the present invention.

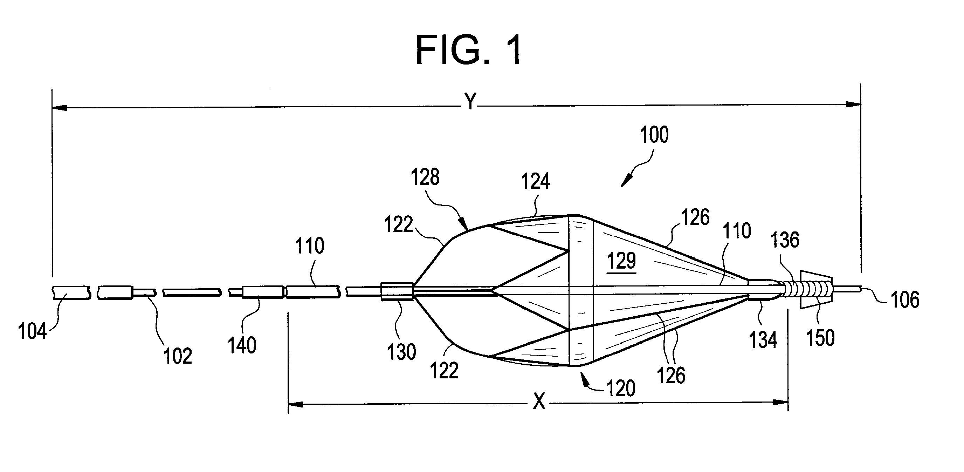

Referring to FIG. 1, embolic protection device **100** comprises a guidewire core **102** having a proximal end **104** and a distal end **106**. Guidewire core **102** is of sufficient length to enable the user of the embolic protection device to route the device to a position in the lumen of a vessel where embolic protection is desired, such as distal to an area of plaque for which an angioplasty procedure is to be used to open the vessel. Typical lengths, y, of the guidewire core **102** are between 150 to 200 centimeter for rapid-exchange systems and between 280 and 330 cm over-the-wire systems but other lengths could be used depending on the application. Guidewire core **102** is metal, preferably stainless steel or another type of non-reactive metal or metal alloy with or without a lubricous coating. The types of lubricious coatings that can be applied to guidewire core **102** include Polytetrafluoroethylene ("PTFE") coatings, silicone coatings and hydrophilic coatings.

A tubular member **110** may be placed over a portion of guidewire core **102** near its distal end **106**. Tubular member **110** is a polymeric tube or coil wire tube. In a preferred embodiment a coil wire tube from Asahi Intec Co., Ltd. is used. Tubular member **110** has a diameter slightly larger than the diameter of the core guidewire core **102** at the distal end. The tolerance between the guidewire core **102** and the tubular member **110** is preferably approximately 0.025 mm (0.001 inch) but could be 0.013 mm (0.0005 inch) or greater. The tubular member **110** can also be coated with a lubricious coating, such as PTFE, internally and/or externally if desired.

For a tubular member **110** that is a coil wire tube the ends are preferably soldered to prevent the wire from unraveling. Also, the ends of the tubing can be ground down a small amount if necessary to approximately 50% of the original wall thickness.

Tubular member **110** may also be composed of a polymer polymeric blend or a polymer fabric. This would give the same torque characteristics, but would results in less stiffness of the distal end. Polymers that could be used for tubular member **110** include PEEK ^{™} polymer from Victrex plc, polyamide, polyurethane, nylon PTFE and polyethylene, among others. VICTREX PEEK polymer, is a repeat unit that comprises of oxy-1, 4-phenylenoeoxy-1, 4-phenylene-carbonyl-1, 4-phenylene. PEEK is a linear aromatic polymer that is semi-crystalline.

Tubular member **110** floats freely on the guidewire core **102** and is not attached to the guidewire core at any point along its length. Tubular member **110** is of a length, x, that is between approximately 10 and 40 centimeters for a rapid exchange system that would be between 150 to 200 centimeters in length. Preferably tubular member should have a length between 15 and 35 centimeters for such a rapid exchange system. Tubular member **110** should have a length between approximately 15 and 35 centimeters for an over-the-wire system that would be between approximately 280 and 330 centimeters in length. The important attribute of the present invention is that the tubular member **110** extend not only under the proximal end of filter **120** but that it extends a substantial distance proximal the filter **120**. Preferably tubular member **110** extends at least approximately 15 centimeters along the guidewire core **102** in the proximal direction. Too long of a tubular member will unnecessarily add to the cost of the device without providing significant added benefit. Additionally, a significantly longer tubular member **110** will result in reduced pushability.

Tubular member **110** may also be significantly shorter at its proximal end or may be left out altogether provided that the longitudinal motion limiter **140** is moved near the proximal end of a shorter tubular member **110** or near the proximal end of the proximal collar **130** of filter frame **128.** If there is no tubular member **110** then the interior diameter of proximal collar **120** and distal collar **134** should be sized so as to float freely on guidewire core **102**. Alternatively, filter frame **128** may be fixed to the guidewire core **102** through any known means such as soldering, brazing, adhesive bonding or other fixation means. Fixation of the filter frame **128** will prevent rotation of the filter frame around guidewire core **128** and obviates the need for longitudinal motion limiter **140.**

A longitudinal motion limiter **140** is a polymer sleeve or other motion limiting device attached to the core guidewire core **102** to prevent migration of the tubular member **110** toward the proximal end of the guidewire core **102.** Attachment of the polymer sleeve may be by any appropriate bonding means. Alternatively, guidewire core may be tapered at this point to transition to a larger diameter at the proximal end of the distal portion in order to prevent such migration.

Filter **120** in FIG. 1 is depicted as having a filter frame **128** having a plurality of proximal struts **122** connected at the proximal end to proximal collar **130** and at the distal end to apposition member **124.** The number of proximal struts is not critical to the invention, but preferably there are at least two proximal struts and more preferably four proximal struts. Apposition member **124** is used to provide proper apposition of the filter **120** against the wall of the vessel. Apposition member **124** is also connected to a plurality of distal struts **126** which are connected to a distal collar **130.** Preferably there are twice as many apposition members **124** as there are proximal struts **122** and an equal number of proximal struts and distal struts. Common strut configurations are 4-8-4 and 6-12-6 although other configurations could be used. Filter frame **128** may be constructed by cutting a single tube of material or may be constructed from a number of different combinations of proximal and distal struts and an apposition member, proximal collar and distal collar which may be interconnected by a number of known means such as welding, brazing or chemical bonding. Filter frame **128** may be made of any number of metal alloys but is preferably made from a "shape memory" metal such as nitinol.

Filter membrane **129** is attached to filter frame **128** and may be comprised of a polymeric blend or may also be made of a thin film of nitinol or other similar metal exhibiting memory characteristics. If filter membrane **129** is a polymeric membrane it can be bonded to filter frame **128** or can be molded around the filter frame **128** as part of the manufacturing process. Filter membrane **129** contains a plurality of holes which are sized to provide a path for fluid flow but are not large enough to permit emboli to pass. The preferred hole size is 0.1 mm (0.00394 inch) and there are preferably approximately 3100 holes per square centimeter.

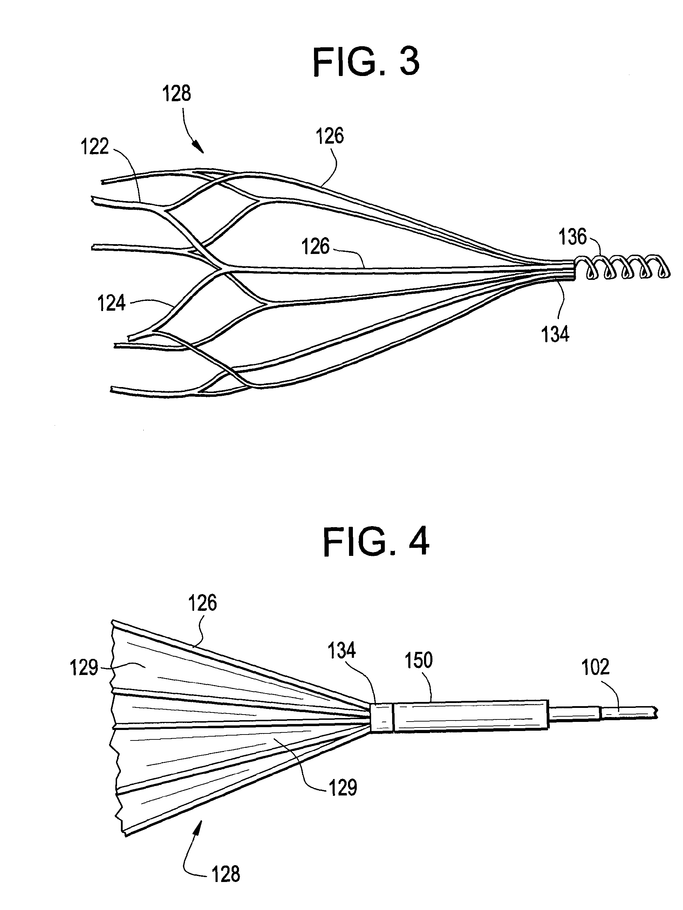

The distal end of filter frame **128** ends in a proximal collar **134**. As will be discussed in detail herein proximal collar **134** has an integral strain relief element **136** which can have a variety of configurations. Strain relief element **136** provides a tunable amount of flexibility depending on the pattern cut into the element. An optional sleeve **150** is a tapered polymeric sleeve or a tapered coil that provides an atraumatic transition between the distal end **106** of the guidewire core **102** and the filter **120.** Strain relief element may also be covered by a polymeric sleeve **150** as shown in FIG. 4.

Other types of filter elements can be substituted for the filter **120** of FIG. 1 of the present invention without departing from the spirit of the present invention. For example, filter 120 could be a wire mesh filter made of a woven wire fabric wherein the interstices between the metal wires provides the proper filtering function or a thin film nitinol mesh. Additionally filter **120** and/or filter membrane **129** could be comprised of woven polymeric threads. Filter **120** may be a self-actuating filter, i.e., one that expands to oppose the vessel wall upon removal from a restraining sheath or other restraining mechanism or the filter may be one that requires actuation such as through mechanical compression or inflation of an actuation balloon. A self-actuating filter is preferred because lack of an actuator results in a lower profile embolic protection device. Additionally, filter **120** could be a mechanically actuated filter requiring one or more wires, rods or other means of mechanical actuation. Other means of filter actuation are also possible.

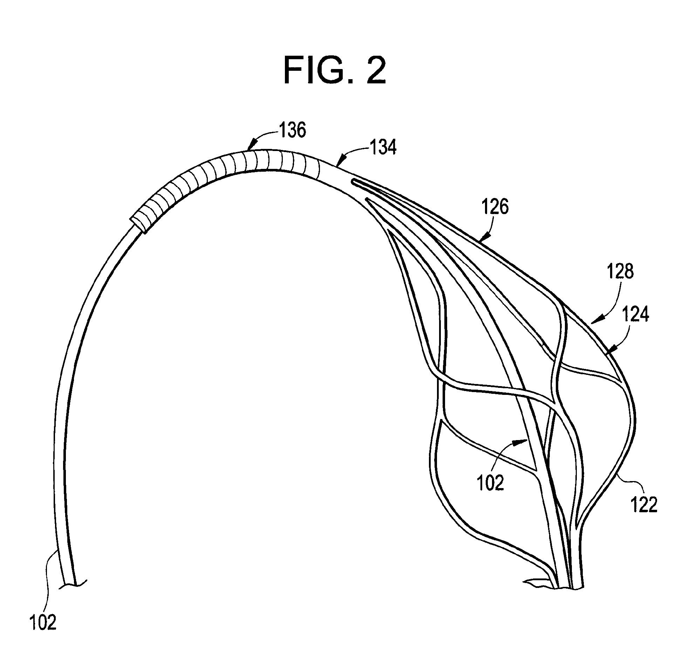

FIG. 2 shows a perspective view of a flexed filter frame **128** (the filter membrane is not shown) having an integrated strain relief element **136** and guidewire core **102**. In FIG. 2 the strain relief element **136** has a double spiral cut pattern. As discussed in greater detail below, the pitch of the spiral cut pattern may be varied in order to change the flexibility of the strain relief element so as to provide the flexibility necessary for a specific application.

FIG. 3 shows an elevational view of a central portion of the filter frame **128** not mounted on a guidewire core and without a filter membrane mounted thereon. Distal collar **134** has at its distal end the integrated strain relief member **136** which is a spiral cut "corkscrew" pattern. The width of the spiraling "corkscrew" element and the pitch of a spiral may be varied in order to produce variations in flexibility in the distal portion of the filter frame **128** in the transition to the distal tip of the guidewire core **102**.

FIG. 4 shows an elevational view of the filter frame **128** having a filter membrane **129** mounted thereon. A portion of distal collar **134** of the filter frame and the strain relief element **136** are covered by a polymeric sleeve **150** providing an atraumatic transition between the distal end of the filter frame **128** and the guidewire core **102**.

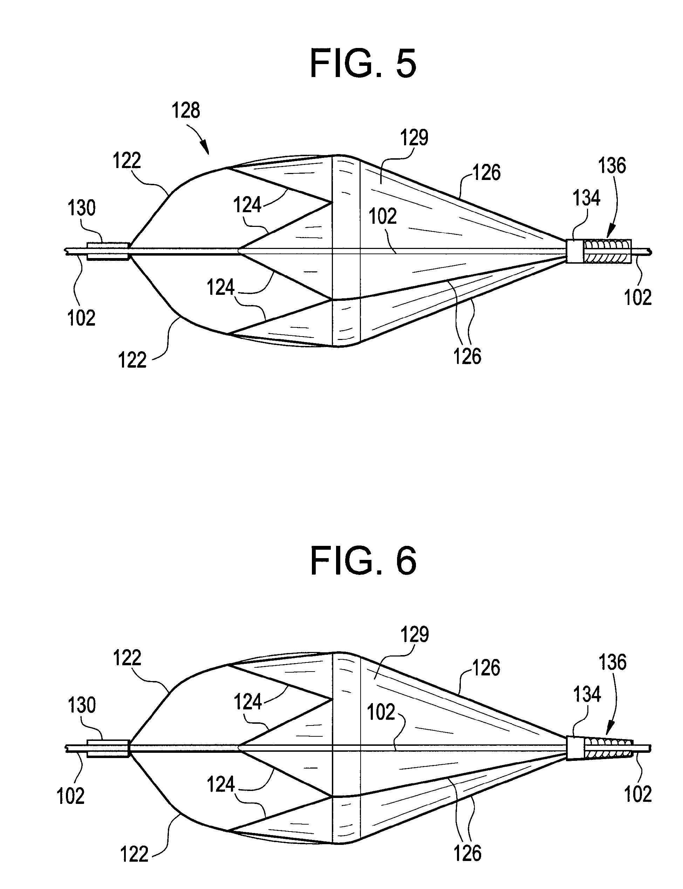

FIGS. 5 and 6 depict two elevational views of a filter element **128** in accordance with the present invention. In FIG. 5 strain relief element **136** is substantially cylindrical and has a double spiral cut pattern while in FIG. 6 strain relief element **136** is tapered along its length toward the distal end of the filter element and also has a double spiral cut pattern. The double spiral cut pattern is advantageous in that it provides symmetric bending properties while also providing an additional safety factor in that for the strain relief to separate from the filter assembly, multiple attachment sites must fracture.

The taper should be between 0.5 degrees and 10 degrees from the longitudinal axis. A tapering distal end will create a smoother transition to guidewire core **102** and will make it easier to install a polymeric sleeve **150** at the distal transition. A tapered design will also provide the filter with the ability to track through calcification or deployed stents without the strain relief element catching on the obstruction, also known as a reduction in the "fish mouthing" of the strain relief.

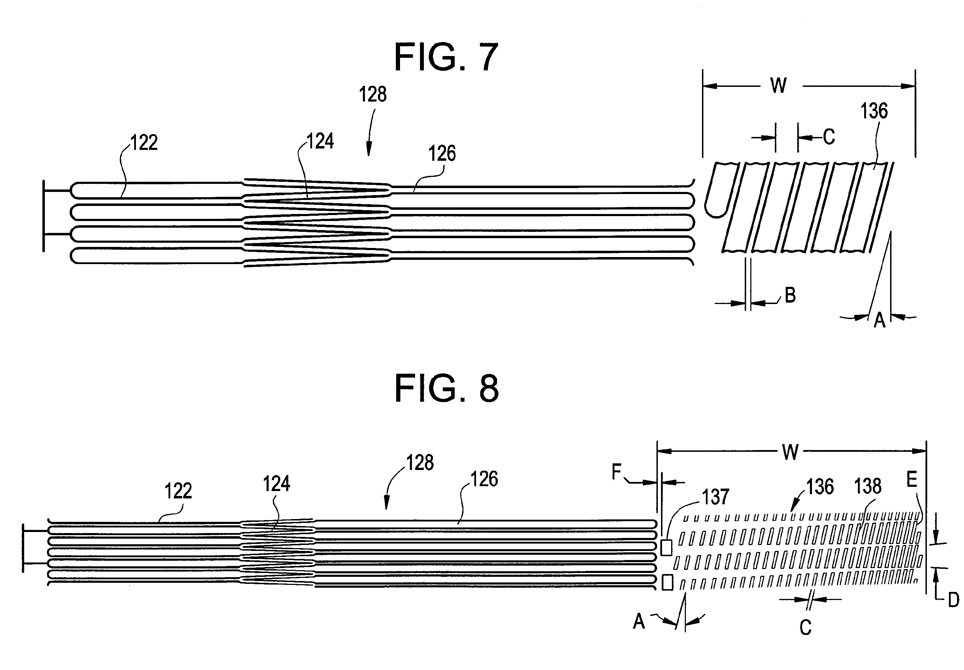

FIGS. 7-12 depict various patterns which can be used to create the strain relief element **136** of 4-8-4 filter frame **128**. FIG. 7 shows a spiral cut pattern having an angle A of approximately 15 degrees where the width of material removed by the double spiral cut (C) is approximately 0.38 mm (0.015 inch) and the width of material remaining (B) is approximately 0.13 mm (0.005 inch). Angle A may be varied to change the characteristics of the strain relief element **136** and should preferable be between 2 and 45 degrees. The ratio of width C to width B should be approximately 3:1, depending on degree of flexibility and rate of transition desired in the strain relief. The width (W) of distal collar **134** and strain relief element 136 together is approximately 3.7 mm (0.146 inch) although the length may vary depending on the strain relief characteristics desired.

In FIG. 8 the cut pattern for a 6-12-6 filter frame **128** is shown. Strain relief element **136** has a plurality of slots **138** made in a spiraling slot pattern with angle A starting at approximately 18 degrees and decreasing to approximately 12 degrees at the distal end at a rate of approximately 0.2 degree every one (1) spiral. Alternatively, angle A may also be kept at a constant 18 degrees. Slots at the distal end have a length E of approximately 0.66 mm (0.026 inch) and decrease in length as the spacing between spirally adjacent slots increases. Angle D is approximately 2 degrees. The width C of each slot **138** is approximately 0.076 mm (0.003 inch). At the distal collar **134** a plurality of rectangular slots **137** are cut around the circumference with each slot having a width of approximately 2.5 mm (0.10 inch) and a height of 3.8 mm (0.015 inch). The slots are an optional feature to ease in the manufacture and handling of the dipped filter membrane and also provide potential device crimping locations for marker bands etc. Width F at the proximal end of the distal collar is approximately 0.13 mm (0.005 inch). The width (W) of distal collar **134** and strain relief element **136** together is approximately 7.8 mm (0.308 inch) although the length may vary.

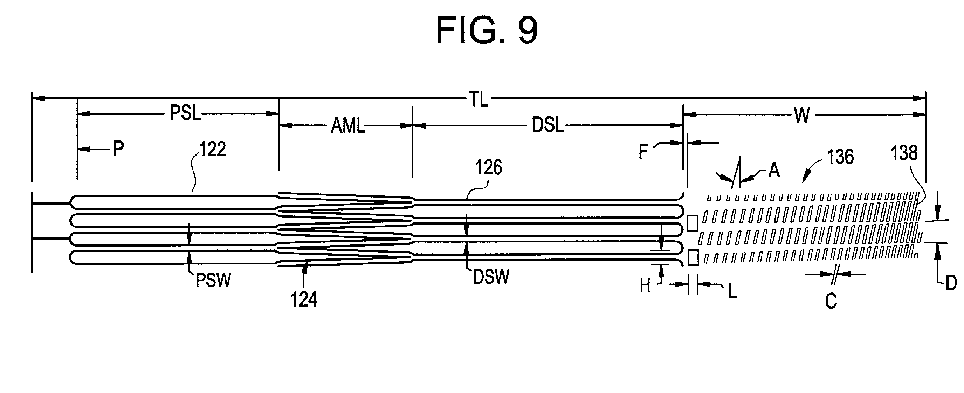

Figure 9 is identical to Figure 8 in the strain relief section of the drawing. The difference is in the filter section proximal to the strain relief i.e. a 6-8-6 filter design versus a 4-8-4 filter design.

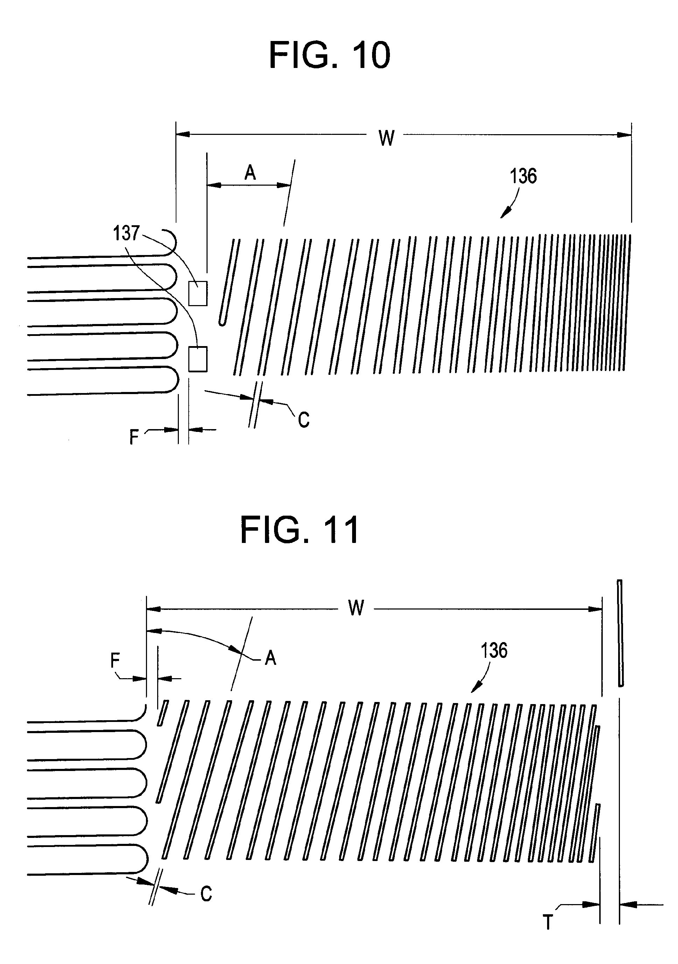

An alternative cut pattern for distal strain relief element **136** is shown in FIG. 10. In FIG. 10 the width C of the spiral of material cut from the tube of material is approximately 0.076 mm (0.003 inch). Angle A is approximately 10 degrees at the proximal end of the strain relief element and is reduced by about 1 degree every third spiral. At the distal collar **134** a plurality of rectangular slots **137** are cut around the circumference with each slot having a width of approximately 0.25 mm (0.010 inch) and a height of 0.38 mm (0.015 inch). Width F at the proximal end of the distal collar is approximately 0.2 mm (0.008 inch). The width (W) of distal collar **134** and strain relief element **136** together is approximately 6.0 mm (0.237 inch) although the length may vary.

An alternative cut pattern for distal strain relief element **136** is shown in FIG. 11. In FIG. 11 the width C of the spiral of material cut from the tube of material is approximately 0.025 mm (0.001 inch). Angle A is approximately 10 degrees at the proximal end of the strain relief element and is reduced by about 1 degree every third spiral. Width F at the proximal end of the distal collar is approximately 0.15 mm (0.006 inch). A distal tip of material of width (T) is left uncut at the distal end of strain relief element 136. In FIG. 11 width T is 0.25 mm (0.010 inch). This uncut material at the distal tip reduces the possibility of a portion of the spiral fracturing particularly when width C is narrow. The width (W) of distal collar **134** and strain relief element 136 together is approximately 6.1 mm (0.241 inch) although the length may vary.

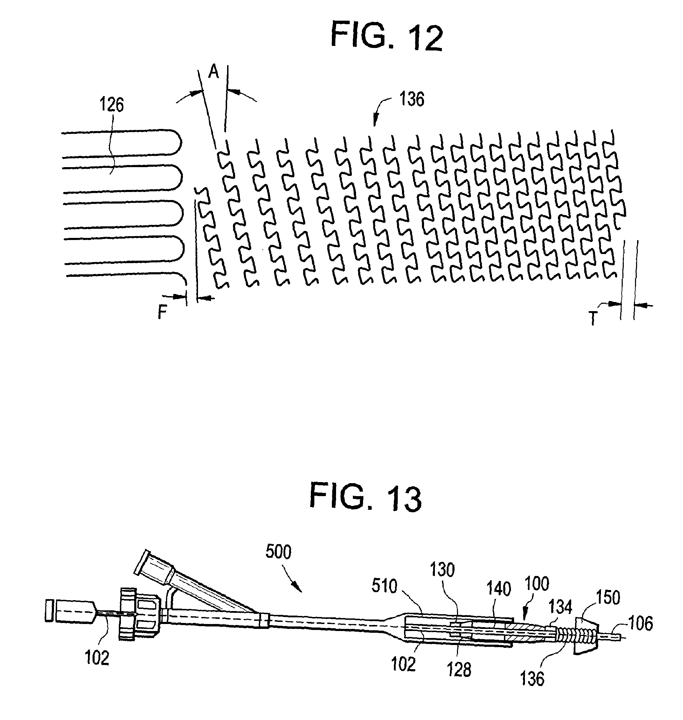

An alternative cut pattern for distal strain relief element **136** is shown in FIG. 12. In FIG. 12 a castellated cut pattern creates a series of interlocking elements. Angle A is approximately 12 degrees at the proximal end of the strain relief element and is reduced by about 0.6 degree every third spiral. Width F at the proximal end of the distal collar is approximately 0.2 mm (0.008 inch). A distal tip of material of width (T) is left uncut at the distal end of strain relief element **136**. In FIG. 12 width T is 0.2 mm (0.008 inch). The width (W) of distal collar **134** and strain relief element **136** together is approximately 6.1 mm (0.242 inch) although the length may vary.

The filter frame **128** is typically made of a nickel-titanium (Nitinol) alloy cylinder having a thickness of between 0.05 mm (0.002 inch) and 0.25 mm (0.010 inch). The various patterns for strain relief as well as the struts of the filter frame are cut into the cylinder using a laser. Integration of the strain relief reduces the manufacturing steps of making a separate component for the strain relief and then attaching the strain relief element to the distal collar of the filter frame. The spiral is formed by laser cutting and then is heat set around a mandrel of suitable diameter causing the remaining material to form (within limits) to the outer diameter of the mandrel. This provides the required inner diameter of the distal (or proximal) leg. The shape could be changed to form a gradual taper by using a tapered mandrel.

FIG. 13 depicts an embodiment of an embolic protection filter system in accordance with the present invention. In FIG. 13 the embolic protection device **100** is placed within a deployment and delivery system **500** that comprises a distal portion **510** coaxially disposed around the guidewire core **102** and the filter **120** with filter frame **128,** proximal collar **130,** distal collar **134,** strain relief element and tubular member **110**. Narrower proximal portion **508** of delivery system **500** is disposed around guidewire core **102.** In use the deployment and delivery system **500** with an embolic protection device **100** contained therein is placed through an incision in the patient into the lumen of a vessel of the patient. Once the distal end of the embolic protection filter system is placed distally of the region of interest and/or treatment such as a lesion, the filter is uncovered and the filter basket radially expands to oppose the lumen of the vessel of the patient. Upon completion of the procedure at the treatment site, the filter is collapsed using a retrieval catheter (not shown) as is known in the art. The embolic protection filter system is then removed from the vessel of the patient.

The preceding description has been presented with reference to presently preferred embodiments of the invention. Workers skilled in the art and technology to which this invention pertains will appreciate that alterations and changes in the described structure may be practiced without meaningfully departing from the scope of this invention, which is defined in the appended claims.

## Claims

1. An embolic protection device for filtering emboli in a fluid stream of a vessel of a patient comprising:
a guidewire core having a proximal end and a distal end and a distal portion extending a first distance from the distal end;
a filter having a filter frame with a distal collar and a filter membrane wherein the filter is mounted near the distal end of the guidewire core; and,
wherein the filter frame has a means for providing strain relief near the transition from the filter and the guidewire core.

2. The embolic protection device of claim 1 wherein the means for providing strain relief is at least one spiral cut through a portion of the distal collar of the filter frame.

3. The embolic protection device of claim 2 wherein the spiral cut through the distal collar of the filter frame has a angle of between approximately 2 and 45 degrees.

4. The embolic protection device of claim 3 wherein the pitch of the spiral cut through the distal collar of the filter frame varies from the proximal start of the cut to the distal end of the cut.

5. The embolic protection device of claim 2 wherein the means for providing strain relief is a double spiral cut through a portion of the distal collar of the filter frame.

6. The embolic protection device of claim 5 wherein the double spiral cut through the distal collar of the filter frame has a angle of between approximately 2 and 45 degrees.

7. The embolic protection device of claim 6 wherein the pitch of the double spiral cut through the distal collar of the filter frame varies from the proximal start of the cut to the distal end of the cut.

8. The embolic protection device of claim 1 wherein the means for providing strain relief is a plurality of slots cut in a portion of the distal collar of the filter frame.

9. The embolic protection device of claim 8 wherein the plurality of slots are cut in a spiral pattern.

10. The embolic protection device of claim 9 wherein the pitch of the spiral pattern of slots cut through the distal collar of the filter frame varies from the proximal start of the cut to the distal end of the cut.

11. The embolic protection device of claim 1 wherein the means for proving strain relief is a castellated cut through a portion of the distal collar of the filter frame creating an interlocking pattern.

12. The embolic protection device of claim 11 wherein the castellated cut is in a spiral pattern.

13. The embolic protection device of claim 12 wherein the pitch of the spiral pattern of the castellated cut through the distal collar of the filter frame varies from the proximal start of the cut to the distal end of the cut.

14. The embolic protection device of claim 1 wherein the embolic protection device further comprises a polymeric sleeve disposed over the means for providing strain relief.

15. The embolic protection device of claim 1 wherein the means for proving strain relief is tapered from its proximal end to its distal end.
